# EUROPEAN PATENT SPECIFICATION

(11) **EP 2 079 758 B1**
(45) Date of publication and mention of the grant of the patent: **30.07.2014**
(21) Application number: 07821242.0
(22) Date of filing: 12.10.2007
(51) Int. Cl.: C07K 14/47, C07K 14/245

(54) **METHOD FOR THE PURIFICATION OF DNAK**
VERFAHREN ZUR AUFREINIGUNG VON DNAK
PROCÉDÉ DE PURIFICATION DE DnaK

(30) Priority: 13.10.2006 EP 06122292; 23.10.2006 US 853567 P
(43) Date of publication of application: 22.07.2009
(73) Proprietor: Biotech Tools S.A., 1120 Brussels (BE)
(72) Inventor: HENOT, Frédéric, 1040 Brussels (BE); LEGON, Thierry, 3360 Bierbeek (BE); PIROTTON, Sabine, 1070 Brussels (BE); PLACIER, Gael, 1030 Brussels (BE)
(74) Representative: Von Kreisler Selting Werner - Partnerschaft von Patentanwälten und Rechtsanwälten mbB
(86) International application number: PCT/EP2007/060875
(87) International publication number: WO 2008/043832

(56) References cited:
- US-A1- 2002 119 163
- ZYLICZ M ET AL: "PURIFICATION AND PROPERTIES OF THE ESCHERICHIA-COLI DNA-K REPLICATION PROTEIN" JOURNAL OF BIOLOGICAL CHEMISTRY, vol. 259, no. 14, 1984, pages 8820-8825, XP002409163 ISSN: 0021-9258
- HENTZ N G ET AL: "Bifunctional fusion proteins of calmodulin and protein A as affinity ligands in protein purification and in the study of protein-protein interactions." ANALYTICAL CHEMISTRY. 15 NOV 1996, vol. 68, no. 22, 15 November 1996 (1996-11-15), pages 3939-3944, XP002409164 ISSN: 0003-2700
- SHERMAN MICHAEL Y ET AL: "Heat shock of Escherichia coli increases binding of dnaK (the hsp70 homolog) to polypeptides by promoting its phosphorylation" PROCEEDINGS OF THE NATIONAL ACADEMY OF SCIENCES OF THE UNITED STATES OF AMERICA, vol. 90, no. 18, 1993, pages 8648-8652, XP002409165 ISSN: 0027-8424
- MÉNORET A: "Purification of recombinant and endogenous HSP70s" METHODS 2004 UNITED STATES, vol. 32, no. 1, 2004, pages 7-12, XP004473395 ISSN: 1046-2023
- NICOLL WILLIAM S ET AL: "Approaches to the isolation and characterization of molecular chaperones" PROTEIN EXPRESSION AND PURIFICATION, vol. 46, no. 1, March 2006 (2006-03), pages 1-15, XP002409167 ISSN: 1046-5928
- UDONO H ET AL: "Hsp-antigen fusion and their use for immunization" METHODS 2004 UNITED STATES, vol. 32, no. 1, 2004, pages 21-24, XP004473397 ISSN: 1046-2023
- NANDAN D ET AL: "A rapid, single-step purification method for immunogenic members of the hsp 70 family: Validation and application" JOURNAL OF IMMUNOLOGICAL METHODS 1994 NETHERLANDS, vol. 176, no. 2, 1994, pages 255-263, XP002409169 ISSN: 0022-1759 cited in the application
- WELCH W J ET AL: "Rapid purification of mammalian 70,000-Dalton stress proteins: Affinity of the proteins for nucleotides" MOLECULAR AND CELLULAR BIOLOGY 1985 UNITED STATES, vol. 5, no. 6, 1985, pages 1229-1237, XP009028111
- MARTINEZ J ET AL: "HSP60, HSP70 and HSP90 from Trichinella spiralis as targets of humoral immune response in rats" PARASITOLOGY RESEARCH 2001 GERMANY, vol. 87, no. 6, 2001, pages 453-458, XP002409170 ISSN: 0932-0113
- GROSS M ET AL: "Control of protein synthesis by hemin. Purification of a rabbit reticulocyte hsp 70 and characterization of its regulation of the activation of the hemin-controlled eIF-2([alpha]) kinase" JOURNAL OF BIOLOGICAL CHEMISTRY 1994 UNITED STATES, vol. 269, no. 36, 1994, pages 22738-22748, XP002409171 ISSN: 0021-9258
- MENORET A ET AL: "Purification of multiple heat shock proteins from a single tumor sample" JOURNAL OF IMMUNOLOGICAL METHODS 03 APR 2000 NETHERLANDS, vol. 237, no. 1-2, 3 April 2000 (2000-04-03), pages 119-130, XP004192500 ISSN: 0022-1759
- PENG P ET AL: "Purification of immunogenic heat shock protein 70-peptide complexes by ADP-affinity chromatography" JOURNAL OF IMMUNOLOGICAL METHODS 1997 NETHERLANDS, vol. 204, no. 1, 1997, pages 13-21, XP002130144 ISSN: 0022-1759 cited in the application
- GROSSMANN M E ET AL: "Proteomics shows Hsp70 does not bind peptide sequences indiscriminately in vivo" EXPERIMENTAL CELL RESEARCH 01 JUL 2004 UNITED STATES, vol. 297, no. 1, 1 July 2004 (2004-07-01), pages 108-117, XP002409174 ISSN: 0014-4827 cited in the application
- MENG S-D ET AL: "Three-step purification of gp96 from human liver tumor tissues suitable for isolation of gp96-bound peptides" JOURNAL OF IMMUNOLOGICAL METHODS 01 JUN 2002 NETHERLANDS, vol. 264, no. 1-2, 1 June 2002 (2002-06-01), pages 29-35, XP004362065 ISSN: 0022-1759
- YOSHIMUNE K ET AL: "Hsc62, a new DnaK homologue of Escherichia coli" BIOCHEMICAL AND BIOPHYSICAL RESEARCH COMMUNICATIONS 08 SEP 1998 UNITED STATES, vol. 250, no. 1, 8 September 1998 (1998-09-08), pages 115-118, XP002409176 ISSN: 0006-291X
- HINODE D ET AL: "A general procedure for the isolation of heat-shock proteins from periodontopathogenic bacteria" JOURNAL OF MICROBIOLOGICAL METHODS 1996 NETHERLANDS, vol. 25, no. 3, 1996, pages 349-355, XP002409180 ISSN: 0167-7012
- JINDAL S ET AL: "Human stress protein hsp70: Overexpression in E. coli, purification and characterization" BIO/TECHNOLOGY 1995 UNITED STATES, vol. 13, no. 10, 1995, pages 1105-1109, XP001247045 ISSN: 0733-222X cited in the application
- EREMEEVA M E ET AL: "Western blotting analysis of heat shock proteins of Rickettsiales and other eubacteria" FEMS MICROBIOLOGY LETTERS 1998 NETHERLANDS, vol. 167, no. 2, 1998, pages 229-237, XP002409181 ISSN: 0378-1097
- TOKUNAGA M ET AL: "Purification and characterization of BiP/Kar2 protein from Saccharomyces cerevisiae" JOURNAL OF BIOLOGICAL CHEMISTRY 1992 UNITED STATES, vol. 267, no. 25, 1992, pages 17553-17559, XP002409196 ISSN: 0021-9258

## Description

The present invention relates to a method for the purification of Heat Shock Proteins.

The expression of Heat Shock Proteins (HSPs) can be upregulated by all cells under conditions of stress. They are molecular chaperones and are involved in protein folding.

HSPs are classified in about six families and highly conserved throughout evolution.

Complexes between HSPs and peptides play a role in antigen presentation. HSPs are also involved in some autoimmune diseases. For a review, see van Eden et al., Nature Reviews. Immunology 5 (2005) 318-330.

DnaK is the bacterial member of the family HSP 70. Methods for the purification of DnaK are known, but,the purity of the preparations is questionable. Typically, DnaK preparations are considered pure, if only one band is detectable in SDS-PAGE. Nevertheless, such preparations are not very pure.

Schönfeld et al. J. Biol. Chem. 270 (1995) 2183 -2189 describe an SDS-PAGE pure DnaK, but the gel filtration analysis shows at least three peaks.

Methods for purification of HSP are known, see for example
- Nandan et al. J., Immunological Methods (1994) 176: 255-264,
- Grossmann et al., Exp. cell Res. (2004) 297: 108-117,
- Peng et al., J. Immunological Methods (1997) 204: 13-21 and
- Jindal et al., Biotechnology (1995) 13: 1105-1109.

Inconsistent data have been reported in the past regarding the immunological properties of HSP, especially DnaK. DnaK enhances the processing of antigens through the Major Hiscompatibility Complex (MHC) of class I or class II on antigen presenting cells (Tobian et al., J. Immunological 172 (2004), 5277-5286; Tobian, Canaday, Harding J. Immunol. 173 (2004) 5130-5137). Native DnaK, depending on their origin, the dosage and the route of administration, can display different, even contradictory, types of immunological effects (van Eden, van der Zee and Prakken Nat Rev. Immunol. 5 (2005): 318-330). Autoimmune responses against autologous HSP have been observed in chronic inflammatory diseases, such as rheumatoid arthritis, type I diabetes and atherosclerosis. Antigenic cross-reaction between bacterial HSP and autologous HSP are suspected to be the cause of autoimmunity development. On the other hand, in clinical trials in type I diabetes and rheumatoid arthritis, HSP have also been shown to promote a switch from a pro-inflammatory cytokine-secretion profile T cell to a regulatory cytokine secretion profile, suggesting an immunoregulation of the inflammatory disease (Bloemendal et al., Clin. Exp. Immunol. 110 (1997): 72-78). More recently, Galdiero et al. have reported that DnaK does not induce the increase of expression of costimulatory molecules (CD80/CD86) on lymphocytes and macrophages (Galdiero et al., Int. J. Immunopathol. Pharmacol. 18 (2005) 637-644).

It is believed that these inconsistencies are based on different purification methods yielding DnaK in combination with various impurities and contaminants. Moreover, since DnaK is an ATPase, it binds adenine nucleotides. Different forms of DnaK coexist and can be purified: DnaK loaded with ATP, DnaK loaded with ADP and DnaK free of nucleotides. These different forms of DnaK could have different immunological effects.

It is the object of the present invention to provide a method for the purification of DnaK overcoming at least some of the drawbacks of prior art, especially providing DnaK of increased purity.

The problem is solved in one aspect by a recombinant purified DnaK preparation, obtainable by a method comprising the steps of
a) ion exchange chromatography,
b) hydroxylapatite chromatography, and
c) gelatin chromatography,
wherein the Dnak preparation has:
- an ATPase activity without the addition of any other chaperone protein,
- a purity of 98 % by weight of protein, and
- is essentially
free of T-cell stimulating impurities.

The purified recombinant heat shock protein is characterized by its ATPase activity without the addition of any other chaperone protein. A suitable test method for ATPase activity is described in the examples.

Furthermore, the preparation is free of T-cell stimulating impurities. This requires a very low endotoxin content. The preparation does not show an effect on TH-1 (production of interferon-y) and TH-2 (IL-5 or IL-13 production). This is further described in the examples.

The preparation is preferably free of immuno-stimulating impurities, including T-cell-stimulating impurities.

"Essentially free of immunostimulating impurities" means:
- no T-cell proliferation is observed up to 30 µg/ml
- no TNF-α production is observed up to 10 µg/ml.

The recombinant purified DnaK preparation has:
a) a residual DNA contamination ≤ 1 ng/mg of protein
b) a residual host cell protein contamination (HCP) < 5% by weight, and
c) an endotoxin contamination < 0.5 E.U./µg of protein.

The purity of the purified DnaK is calculated on a "weight per protein" basis, i.e. at least 98% by weight of protein.

Preferably this value is determined by analysing of an SDS-PAGE gel followed by coomassie staining and densitometry.

The content of DNA is preferably very low, i.e. ≤ 1 ng/mg of protein, preferably ≤ 0.5 ng/mg of protein. The DNA contamination is measured using RT-PCR using specific primers.

The test condition depends on the type of expression system. For example for expression in *E. coli.,* specific primers for 23S DNA are suitable. For *P. pasteuris,* suitable primers are selected in the 18S DNA. These method are known to a person skilled in the art.

Furthermore, the residual host cell protein contamination is < 5% by weight, preferably < 1% by weight, more preferably ≤ 0.0001% by weight. Preferably this is determined in an ELISA test, commercially available from Cygnus Technologies Inc., USA, under the product name "Kit *E.coli* host cell proteins", for measuring *E. coli* cell proteins. The test system must be selected corresponding to the expression system, i.e. Cygnus Technologies Inc. has also developed corresponding ELISA for other expression systems. As an alternative other methods such as SDS-PAGE with silver staining, HPLC or Western Blotting are suitable.

The endotoxin contamination is < 0.5 E.U./µg of protein as shown by LAL kinetic test, commercially available from Cambrex Corporation, USA, under the product name "Kinetic-QCL^{®}. Preferably the content is < 0.1 E.U./µg of protein and preferably < 0.01 E.U./µg of protein.

It has now been understood that DnaK may have contaminations with immunostimulating impurities, especially
- proteins,
- peptides,
- nucleic acid,
- lipopolysaccharides (LPS).

DnaK is a chaperone protein and, therefore, binds to other proteins and peptides.

Purification methods of prior art were obviously not able to remove both, bound and unbound impurities in DnaK preparations. Surprisingly, the purification procedure of the invention makes it possible to obtain a highly pure recombinant DnaK, essentially free of immunostimulating impurities.

For use in pharmaceutical preparations, especially as an adjuvant, it is absolutely necessary that the DnaK does not contain immunostimulating impurities.

A further aspect of the invention is a method for the purification of a recombinant heat shock protein, preferably DnaK from a cell lysate comprising the steps of
a) ion exchange chromatography,
b) hydroxylapatite chromatography, and
c) gelatine chromatography.

In a preferred aspect of the invention, the DnaK is from saprophytic bacteria such as *E. coli* or pathogenic bacteria, such as *Mycobacterium tuberculosis.*

In a preferred embodiment, the ion exchange chromatography is an anion exchange chromatography.

For the hydroxylapatite chromatography, a hydroxylapatite type II chromatography is preferred.

In one embodiment, the gelatin chromatography is conducted on gelatin sepharose. The DnaK is preferably desorbed from the gelatin using a nucleotide, e.g. ADP, ATP.

A further aspect of the invention is a method for forming a complex between recombinant DnaK and at least one peptide or at least one protein comprising the steps of
a) combining recombinant DnaK of the invention with ATP at a molar ratio DnaK:ATP of 1:1 to 1:10;
b) adding at least one peptide or at least one protein; and
c) incubating at a temperature of 10°C to 60°C, preferably 20°C to 45°C.

A further aspect is a mixture or complex between recombinant purified DnaK of the invention and at least one peptide or at least one protein. Preferably proteins are used in a denaturated form.

The present invention also covers combinations of DnaK with two or more different peptides or the combination of DnaK with several (optionally denaturated) proteins and complexes between DnaK and peptides and (optionally denaturated) proteins.

Suitable peptides are for example insulin, thyroglobulin, thyroid peroxidase, type II collagen, gliadin, GAD65, proteolipid protein, S-antigen, acetylcholin receptor, haptenized colonic proteins, interphotoreceptor retinoid binding protein, myelin basic protein, myelin oligodendrocyte glycoprotein, peripheral nerve P2, cytoplasmic TSH receptor, intrinsic factor, lens proteins, platelets, nucleoproteins such as histones, heat shock proteins, MHC I, MHC II, MHC-peptides complexes, milk allergens, venom allergens, egg allergens, weed allergens, grass allergens, tree allergens, shrub allergens, flower allergens, grain allergens, fungi allergens, fruit allergens, berry allergens, nut allergens, seed allergens, bean allergens fish allergens, shellfish allergens, meat allergens, spices allergens, insect allergens, mite allergens, animal allergens, animal dander allergens, allergens of Hevea brasiliensis, coagulation factors and blood group antigens, vegetables allergens, mould allergens, cytokines, proteins or peptides involved in neurodegenerative diseases (for example Alzheimer), peptides loaded with addiction substances and fragments thereof. These proteins could be used either directly, preferably in a denaturated form or after hydrolysis into smaller fragments.

A further aspect of the invention is the use of the DnaK of the invention in a pharmaceutical composition as a carrier protein, or as an adjuvant inducing an humoral response, a regulatory T-cell response in the absence of any other T-cell response.

A further aspect is the use of the DnaK of the invention or the mixture of the invention or the complex of the invention for *in-vitro* diagnostics and the use of the recombinant purified DnaK of the invention or the complex of the invention for the preparation of a pharmaceutical composition for inducing the tolerance, wherein the tolerance is suitable in the treatment and/or prevention of allergy, autoimmune disease or graft rejection, or neurodegenerative diseases.

In all kinds of application the DnaK of the invention may be used either in complex with ATP, in complex with ADP or free of nucleotides. For some uses it is also useful to hydrolyze the DnaK of the invention. "Free of nucleotides" is understood as less than 5% nucleotide on a molar basis.

Typical autoimmune diseases are inter alias Systemic Lupus erytematosus disease, Sjögren's disease, rheumatoid polyarthritis, as well as pathologies such as sarcoidosis and osteopenia, spondylarthritis, scleroderma, multiple sclerosis, amyotrophic lateral sclerosis, hyperthyroidism, Addison's disease, autoimmune hemolytic anemia, Crohn's disease, Goodpasture's syndrome, Graves' disease, Hashimoto's thyroiditis, idiopathic purpural hemorrhage, insulin-dependent diabetes, myasthenia, pemphigus vulgaris, pernicious anemia, poststreptococcal glomerulonephritis, psoriasis and spontaneous sterility.

The medicament may be administered for example intraveneously, intramusculary, orally, intranasally or intrapulmonary. Preferred ways are sublingual, buccal or entric delivery.

"Sublingual administration" and "buccal administration" are methods wherein the substance is combined in a pharmaceutical formulation which allows absorption of at least one substance in the mouth mucosa. Sublingual administration involves the patients holding a (sublingual) pharmaceutical composition or dosage from under their tongue while the substance diffuses into the mouth, through the mucosa lining the mouth. In buccal administration, the patients hold the (buckle) pharmaceutical composition or dosage from between their cheek and gingiva (gum) instead of under the tongue. The buccal administration can be chewed to allow faster buccal absorption or release; the present invention therefore provides in a preferred embodiment a gum-based formulation or a chewing gum formulation.

"Enteric delivery" is a method wherein the substance is in a pharmaceutical formulation which protects the active ingredient from absorption and/or degradation prior to entry into the intestine. Preferably absorption is effected in the ileum, duodenum or jejunum. In one preferred embodiment, the said pharmaceutical formulation can be a suppository.

Especially suitable formulation includes coating with polymers, e.g. as sold under the trademark Eudragit^{®}, commercially available from Degussa, Germany, or cellulose acetophthalate available from Fagron or hydroxypropyl methylcellulose phthalate available from Shin-Etsu Chemicals Co., Ltd. These polymers are suitable for solid oral formulations which are released in the intestine. In a preferred embodiment, suitable pharmaceutical formulations are comprising any needed binders or excipients for the neutralization of hydrochloric acid (gastric acid secretion) and/or the inhibition of pepsin and/or the stimulation of bicarbonate and mucus secretion in a patient.

Neutralization of hydrochloric acid and/or inhibition of pepsin in the stomach can be achieved for example with sucralfate or a proton-binding polymer, such as but not limited to polyethylenimine, or any neutralizing anti-acid (antacid) or any acid blocker selected from the group consisting of aluminum salts, bismuth salts, magnesium salts, sodium bicarbonate, potassium bicarbonate, potassium citrate, sodium potassium tartrate, tricalcium phosphate, and mixtures thereof.

Some other types of acid blockers that can be used in the suitable formulation are called gastric proton pump inhibitors (or gastric H+/K+ ATPase inhibitors), prostaglandin analogues and histamine H2-receptor antagonists. These include, but are not limited to, misoprostol, ranitidine (used in ZANTAC®), cimetidine (used in TAGAMET®), nizatidine (used in AXID®), famotindine (used in PEPCID®), sufotidine, roxatidine, bisfentidine, tiotidine, lamtidine, niperotidine, mifentidine, zaltindine, loxtidine, omeprazole (used in PRISOLEC®), and rabeprazole.

Preferably, the DnaK is used with bulking agents selected from non-reducing sugars.

In another preferred embodiment, the suitable formulation comprises a microsphere of the said at least one substance bound to or encapsulated in an inert particle in whatever shape or form, having a mesh size of about 30 - 35 mesh (about 600 µm to 500 µm) or greater than about 40 mesh, and most preferably in the range of about 45 to 200 mesh, and may be for example a nonpareil, a silica powder, a salt crystal or a sugar crystal.

### Legends of the figures

**Figure 1****:** SDS-PAGE analysis after Q-sepharose HP chromatography in accordance with example 1.
**Figure 2****:** SDS-PAGE analysis after hydroxyapatite type II chromatography in accordance with example 1.
**Figure 3****:** SDS-PAGE analysis after diafiltration/size exclusive chromatography.
**Figure 4****:** SDS-PAGE analysis after gelatin sepharose chromatography. Samples (5 µg of each preparation) were denaturated and loaded on a 12% Bis/Tris NU-PAGE gel. After electrophoresis, the proteins were stained by Silver staining.
QSHP = Q Sepharose HP; HA = Hydroxyapatite; GS = Gelatin Sepharose.
**Figure 5****:** SDS-PAGE analysis of the purified DnaK. The drug substance is denaturated and loaded on a 4-12% Bis/Tris NU-PAGE gel. Following electrophoresis, the protein was stained with Coomassie blue R250.
**Figure 6****:** ATPase activity of DnaK.
**Figure 7****:** Pro-inflammatory effect of DnaK. Whole blood was incubated in the presence of different concentrations of DnaK or bacterial lipopolysaccharides (LPS). The cytokines interleukin-1b (IL-1b), interleukin-6 (IL-6) and Tumour Necrosis Factor-α (TNFa) were quantitated by ELISA.
**Figure 8****:** Effect of the DnaK on T-cell responses. Human PBMC were incubated 6 days in the presence of DnaK (10 µg/ml) or phytohemagglutinin (5 µg/ml). The different cytokines were quantitated in the culture medium, by ELISA. Results are expressed as mean ± deviation of 2 determinations, for 6 different donnors. CTRL = control.
**Figure 9****:** Effect of DnaK on IFN-γ production induced by candidin. Human PBMC were incubated 3 days in the presence of candidin (3.5 µg/ml) and increasing concentrations of DnaK. IFN-γ production was quantitated by ELISA in the culture medium. Results are expressed as mean ± standard deviation of 4 determinations.
**Figure 10****:** IL-10 production by Treg. Purified CD4⁺/CD25⁺ cells and/or CD4⁺CD25⁻ cells were added in precise amounts to purified dendritic cells pretreated 3 days with DnaK (10 µg/ml) or BSA (10 µg/ml). Cells were further cultured in the presence of DnaK or BSA and were stimulated or not with PHA.
IFN-γ and IL-10 were quantitated in the culture medium by ELISA. 25-/25+ represents the mixing of the 2 populations in a 1/1 ratio.
**Figure 11****:** Presence of anti-DnaK antibodies in human serum. DnaK or BSA were coated in 96 well plates to be used as capture protein for the ELISA test.
Serum from healthy volunteers and allergic patients were used at a dilution of 1/100. IgG were detected with anti-human IgG-HRP antibodies.

The invention is explained in more details by the following examples.

### Example 1: DnaK production and purification

### DnaK production

### Bacterial strain

The *Escherichia coli* strain JS219/pOFXtac-1/KJ1 is genetically modified by the introduction of a plasmid coding for the *E. coli* DnaK. Cloning step and the construction of the vector pOFXtac-1/KJ1 have been described by Castanie MP, Berges H, Oreglia J, Prere MF, Fayet O A set of pBR322-compatible plasmids allowing the testing of chaperone-assisted folding of proteins overexpressed in Escherichia coli; Anal Biochem (1997) 254(1): 150-152.

### Pre-culture

One vial of Master Seed (1.5 ml) is allowed to thaw to room temperature. Three 2 l shake flasks containing 500 ml of YES + kanamycin medium (30g/l yeast extract, 5 g/l NaCl and 50 mg/l kanamycin acid sulfate) are each inoculated with 400 µl of Master seed and incubated under shaking (270 rpm) for 6 ± 0.5 hours at 37°C, until the OD₆₀₀ₙₘ is above 0.7 units

### Fermentation

The volume of pre-culture is added to a pre-sterilised fermentor containing 50 l of NRJ18 + kanamycin medium (KH₂PO₄ 5.2 g/l, K₂HPO₄ 10.7 g/l, glycerol 0.4 g/l, yeast extract 50 g/l, soya peptone 30 g/l, MgSO₄*7H₂O 2.5 g/l, SAG471 0.6 ml/l, kanamycin acid sulfate 100 mg/l). The temperature is maintained at 37°C ± 5°C and the pressure at 360 mbar. When optical density is 25 ± 1, the culture is induced by addition of IPTG (1 mM final concentration). The induction conditions are then maintained for 4 hours. The culture is rapidly cooled below 20°C. The medium is filtered trough a 50 pm Sartopure PP2 filtration device. The cell pellets were recovered by centrifugation at 4500 rpm (5000 x g) for 30 minutes at 4°C and frozen at -20°C until use.

### Cell disruption and supernatant filtration

The cell pellets are thawed at room temperature, in 20 mM Tris-HCl pH 8. Then, the cell paste concentration is adjusted at 166.6 g fresh cell weight / liter by adding buffer. The cell suspension is filled into a tank placed on ice. The homogenised cells are disrupted with a Niro Soavi Panda high-pressure disrupter at an average pressure of 800 & 50 bars, during 2 cycles.

The cell lysate is centrifuged for 30 minutes at 16000 g (8000 rpm), at 4°C. The supernatant is collected and filtered through a Sartobran P Maxicap 10 inches (0.2 µm). The filtered supernatant is kept at 4°C overnight before next step.

### DnaK Purification

### Q Sepharose HP chromatography (QHP)

Anion exchange chromatography is conducted with Q Sepharose HP (Amersham Biosciences). The column is packed in highly purified water at a linear flow rate of 68 cm/h (33.4 l/h). The dimensions of the packed column bed are: diameter = 250 mm, cross-sectional area = 132 cm², bed = 25.0 cm, packed volume = approximately 12.27 l.

The filtered supernatant is diluted twice with 20 mM Tris-HCl pH 8.0 and, if required, the pH is adjusted to pH 8 ±.0.1 with a 1 M Tris solution (unadjusted pH solution). Final conductivity must be < 10 mS/cm. The diluted supernatant is then loaded on the column at a flow rate of 22.1 l/h (45 cm/h). After loading is complete, the column is washed with 2.0 to 3.0 CV of 20 mM Tris-HCL pH 8.0 at 19.6 l/h (41.5 cm/h), until the baseline reaches zero. The elution is performed in 2 steps at 22.1 l/h (45 cm/h): the first step is performed with 2-3 CV of 20 mM Tris-HCl + 0.25 M NaCl pH 8.0 buffer. The second step is performed with 2-3 CV of 20 mM Tris-HCl + 0.45 M NaCl pH 8.0 buffer. The fraction collected during this step is retained for the following purification step. The absorbance is followed at 280 nm. The eluate is stored at 2-8°C overnight until the next purification step.

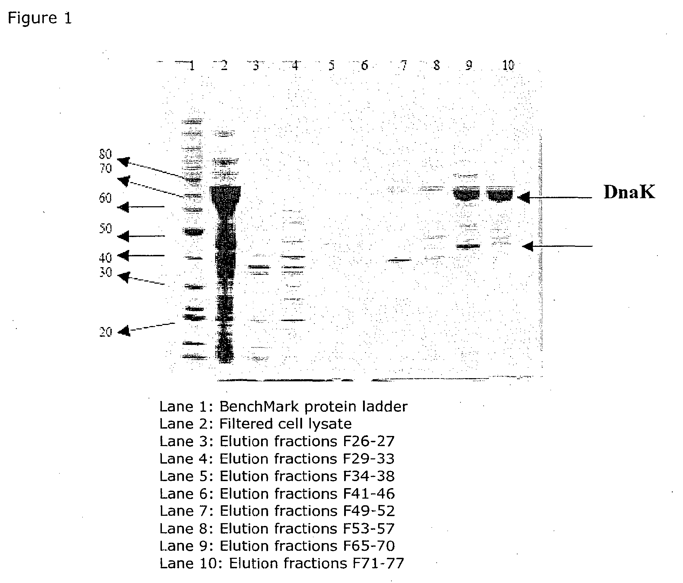

The samples (20 µl of each fraction) were denaturated and loaded on a 4 to 12% Bis/Tris NU-PAGE gel. After electrophoresis, the proteins were stained with Coomassie Blue (see figure 1).

### Hydroxyapatite type II chromatography (HA)

The second chromatography is conducted with Hydroxyapatite type II - 40 µm (Bio-Rad). The resin (2000 g) is poured in 3.2 1 of Na₂HPO₄*12H₂O + NaH₂PO₄*2H₂O 200 mM - pH 6.8 under gentle mixing. The column is packed with Na₂HPO₄*12H₂O+ NaH₂PO₄*2H₂O 200 mM - pH 6.8 at a linear flow rate of 175 cm/h (26.9 l/h). The dimensions of the packed column bed are: diameter = 14 cm, cross-sectional area = 154 cm², bed = 21.5 cm, packed volume = 3.31 l.

The QHP eluate is loaded in 6 equal fractions on the HA column. All the Hydroxyapatite process is performed at a flow rate of 150 cm/h (23.l l/h).

The column is equilibrated with 2.5 to 3.5 CV of 200 mM NaH₂PO₄/ Na₂HPO₄ pH 6.8 followed by 2.5 to 3.5 CV of 5 mM NaH₂PO₄/ Na₂HPO₄ pH 6.8 until pH and conductivity are stabilized.

After pH adjustment to 6.8 ± 0.1 with 50% HCl, the QHP eluate (a sixth of the total volume) is loaded on the column (it's important to notice that the NaCl concentration of the previous chromatographic step is not important for HA adsorption). After loading is complete, the column is washed with 1.5 to 2.5 CV of 5 mM NaH₂PO₄ + Na₂HPO₄ pH 6.8 buffer, until U.V. returns to the baseline. The absorbance is followed at 280 nm.

After that, the elution is performed with a gradient from 0% to 100% of 200 mM NaH₂PO₄ / Na₂HPO₄ pH 6.8 buffer in 10 CV. Usually, the elution is complete after about 6 CV.

The eluted peak is collected, the pool of fractions is filtered through a 0.22 µm filter and kept at 2-8°C.

After elution, a regeneration step is performed with 2.5 to 3.5 CV of 5 mM NaH₂PO₄ + Na₂HPO₄ pH 6.8 buffer, before the loading of the next sample.

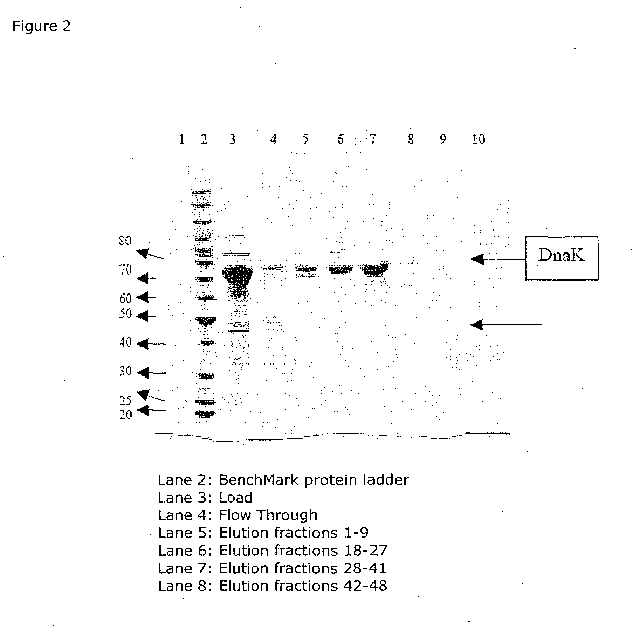

The samples were denaturated and loaded on a 4 to 12% Bis/Tris NU-PAGE gel. After electrophoresis, the proteins were stained with Coomassie Blue (see figure 2).

### Diafiltration/size exclusion chromatography

It was tried to separate the impurities by diafiltration on a 30 kDa membrane and by chromatography on a Sephacryl S100 HR resin. The impurities could not be removed by these two methods. Indeed, the impurity is recovered in the retentate of the diafiltration as DnaK. The impurity was co-eluted with DnaK in the Sephacryl S100 chromatography.

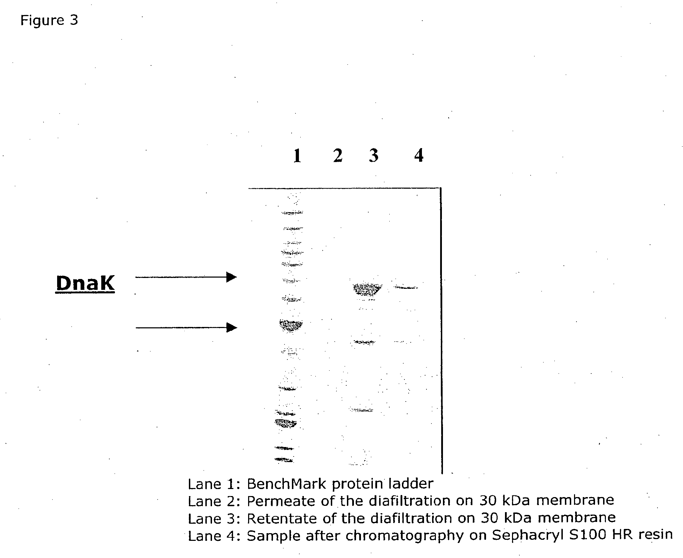

The SDS-PAGE analysis of these different samples is shown in figure 3. The samples were denaturated and loaded on a 4 to 12% Bis/Tris NU-PAGE gel. After electrophoresis the proteins were stained with Coomassie Blue.

### Gelatin Sepharose Fast Flow chromatography (GSFF)

The third method tested to separate impurities from DnaK involves chromatography on Gelatin Sepharose Fast Flow resin (GE Healthcare). The column is packed in highly purified water at a linear flow rate of 150 cm/h (19.9 l/h). The dimensions of the packed column bed are : diameter = 13 cm, cross-sectional area = 133 cm², bed = 15 cm, packed volume = 1.99 l.

The pool of HA eluates is loaded in 9 equal fractions on the GSFF column. In between the GSFF cycles, a cleaning with 6 M Guanidium chloride is performed. All the Gelatin Sepharose process is performed at a flow rate of 120 cm/h (15.9 l/h).

The column is sanitized with 1.5 to 2.5 CV of 70% ethanol + 0,1 M acetic acid with a contact time of 1 hour. It is then equilibrated with 2.5 to 3.5 CV of 0.5 M NaCl + 5 mM HEPES, pH 7.5.

The HA pool of eluates (a ninth of the total volume) is loaded on the column. After loading is complete, the column is washed with 1.5 to 2.5 CV of 0.5 M NaCl + 5 mM HEPES, pH 7.5 buffer, until U.V. returns to the baseline. The absorbance is followed at 280 nm.

After that, the elution is performed with 3 to 4 CV of 0.5 M NaCl + 5 mM HEPES + 3 mM ATP + 1 mM MgCl₂, pH 7.5.

When the absorbance rises, fractions are collected during 2 CV. After the ninth GSFF cycle, the column is cleaned with 1.5 to 2.5 CV of 6 M Guanidium chloride then washed with 3 CV of 0.5 M NaCl + 5 mM HEPES, pH 7.5 buffer and stored in 20% ethanol.

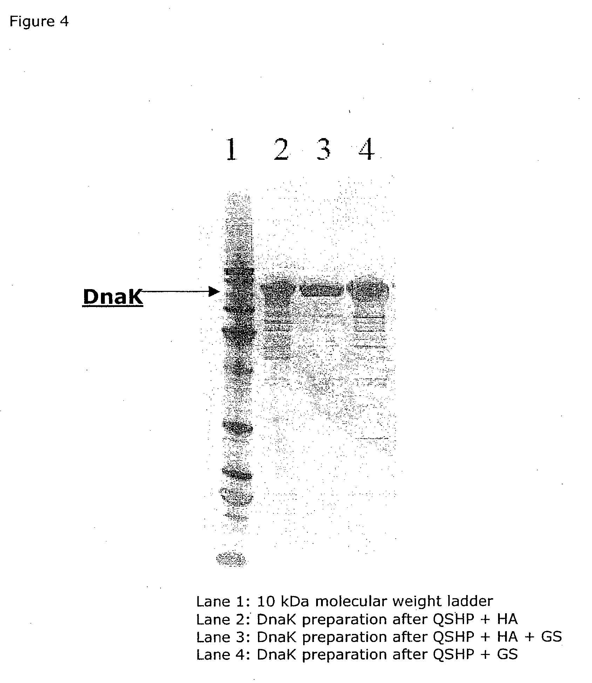

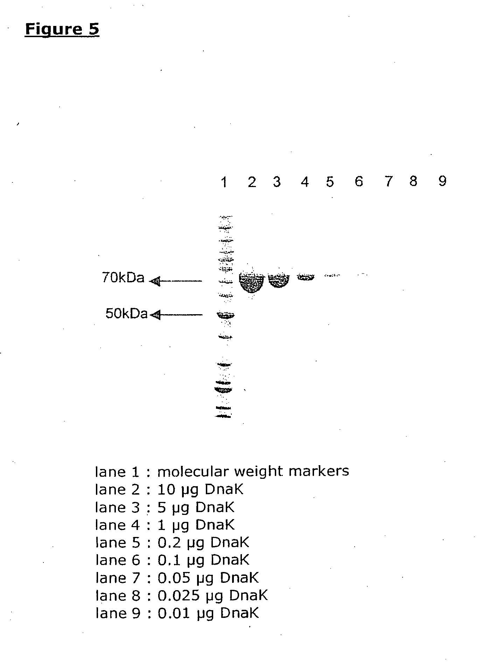

As shown in figure 4 chromatography on Gelatin Sepharose resin clearly improves the purity of the DnaK in comparison to the sample obtained after hydroxyapatite chromatography (comparison of lane 2 and lane 3). In this case, the purity of the DnaK is greater than 98.25% (see example 2 and figure 5).

When the supernatant of cell lysate is directly loaded on a Gelatin Sepharose column, the purity of the DnaK after this step is about 95%.

When the Gelatin Sepharose step is performed after the Q Sepharose HP, the purity of DnaK preparation is not increased in comparison to the sample obtained after Q Sepharose HP and Hydroxyapatite chromatography (figure 4 comparison of lane 2 and lane 4).

These results demonstrate that the combination of these three types of chromatography (IEX, HA and GSFF) is absolutely necessary to obtain a DnaK preparation with purity > than 98%.

### Concentration and diafiltration by tangential flow filtration

Concentration is conducted with two PLCTK pellicon 2 membranes (30 kDa molecular weight cut-off, 0.1 m², Millipore,) using a Proflux M 12 (Millipore).

The membranes are washed twice with 3 l of water for injection and membranes integrity is tested prior to operation. Sanitization is performed by continuous recirculation of 0.5 M NaOH for 60 minutes. The membranes are then rinsed with Na₂HPO₄*12H₂O 30 mM + NaH₂PO₄*2H₂O 20 mM pH 7.3 buffer until the permeate pH reaches 7.3 ± 0.1.

The pooled Gelatin Sepharose eluates (GSFF-E pool) are concentrated to 2000 ml and then diafiltrated against 10 volumes of Na₂HPO₄*12H₂O 30 mM + NaH₂PO₄*2H₂O 20 mM pH 7.3 buffer in order to exchange the buffer. The following process parameters are used : Pᵢₙ= 1.5 ± 0.1 bar, Pout= 0.5 ± 0.1 bar, TMP= 1 bar.

Following the diafiltration, the system and membranes are rinsed twice with 200 ml of Na₂HPO₄*12H₂O 30 mM + NaH₂PO₄*2H₂O 20 mM pH 7.3 buffer. The used rinsing solution is added to the diafiltrated retentate.

Following diafiltration, the system and membranes are rinsed with water for injection. Sanitization is performed by continuous recirculation with sodium hydroxide 0.5 M for 60 minutes. The system is stored in 0.1 M sodium hydroxide.

### Sterile filtration

According to a UV assay performed on the diafiltrated retentate, its concentration is adjusted to 2.5 mg/ml with Na₂HPO₄*12H₂O 30 mM + NaH₂PO₄*2H₂O 20 mM pH 7.3 buffer.

Filtration of the diafiltrated DnaK-ATP fraction from step 4 is performed on a Millipak 60 (0.22 µm filter). Prior to filtration, the filter is rinsed with Na₂HPO₄*12H₂O 30 mM + NaH₂PO₄*2H₂O 20 mM pH 7.3 buffer. All aliquots of DnaK-ATP are stored at -20°C.

### Example 2: DnaK characteristics

The DnaK obtained by example 1 is very pure:
Protein Purity
   - in terms of proteins, the purity is greater than 98.25% as determined by Coomassie staining of the SDS-PAGE gel (figure 5). The substance was denaturated and decreasing quantitites were loaded on a 4-12% Bis/Tris NU-PAGE gel. The proteins were separated by electrophoresis, and stained with Coomassie blue R250 (0.1%). The product purity is assessed by means of comparing the intensity of each possible contaminating bands detectable in lane 2 with the band intensity of the DnaK at different dilutions.
Residual DNA content
   - the residual DNA content, quantified by RT-PCR with bacterial DNA specific primers for 23 S chromosonic DNA, is 0.22 ng/mg of protein. The quantitative real-time PCR is based on the amplification of genomic DNA by the polymerase chain reaction (PCR) method in which the amount of amplified DNA is followed in real time by fluorescence measurement, using the SYBR green dye which binds to newly formed double strand DNA. The primers for bacterial DNA have been chosen in the 23S chromosomic DNA as proposed by Smith et al. (Smith et al., BioTechnique (1999) 26: 518-526).
Residual host cell protein content
   - the residual host cell protein content determined by ELISA is 0.00004%. The bacterial protein content measurement developed by Cygnus Technologies Inc. is based on the principle of sandwish ELISA. Briefly, 96 well plates are coated with capture anti-*E.coli* antibodies. *E.coli* proteins contained in the samples are trapped by these antibodies and detected using other specific *E.coli* antibodies coupled to alkaline phosphatase. After washing to remove unbound reagents, the substrate of the enzyme (p-nitrophenylphosphate - pNPP) is added and the absorbance measurement is proportional to the reaction product concentration and thus to the host cell proteins present in the sample.
Endotoxin content
   the endotoxin content (determined by LAL method) is 0.002 E.U./µg of protein. The method used to quantify the endotoxins is the Kinetic-QCL^{®} test developed by Cambrex. It is based on the following principle that gram negative bacterial endotoxins catalyze the activation of a proenzyme in the lysate of Limulus Amebocyte (LAL). Upon activation of this enzyme, p-nitroaniline (pNA-yellow) is released from Ac-Ile-Glu-Ala-Arg-pNa (colorless). The absorbance at 405 nm is measured continuously during the incubation period and is proportional to the pNA concentration in the medium. The concentration of endotoxin in the sample is calculated from its reaction time by comparison to the reaction time of known amounts of *E.coli* endotoxin standards.
Nucleotide content
   - 40% of the purified DnaK obtained by this method corresponds to DnaK free of nucleotides and at the other 60% are the DnaK-ADP.

### Example 3: ATPase activity of the DnaK

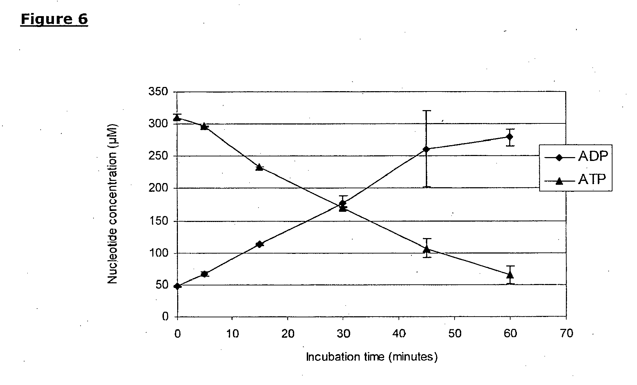

In order to check the functionality of the DnaK, we have measured its ATPase activity. Exogenous ATP was added and the production ADP was followed with time. The nucleotides are analyzed by ion-pairing reverse-phase chromatography on C18 column. The rate of ATP hydrolysis is 0.05 min⁻¹ which is in agreement with the values reported in the literature (Jordan et McMacken, J. Biol. Chem. (1995) 270 : 4563-4569). In detail, exogenous ATP (6 molar equivalents) was added to the DnaK, in the presence of Tris 50 mM, pH 7.4, MgCl₂ 3 mM and KCI 10 mM. An aliquot (500 µl) of the reaction medium was harvested after 5, 15, 30, 45 and 60 minutes and the reactions were stopped by adding 100 µl of HCl 1 M. Nucleotides were separated from DnaK by filtration on 10 kDa filters and after neutralisation of the filtrate, were analysed by ion-pairing reverse-phase chromatography on C18 column (figure 6). The elution solution was composed of 100 mM phosphate buffer and 20% methanol, tetrabutyl ammonium was used as pairing agent.

### Example 4: biological properties of DnaK

### DnaK has no pro-inflammatory effect:

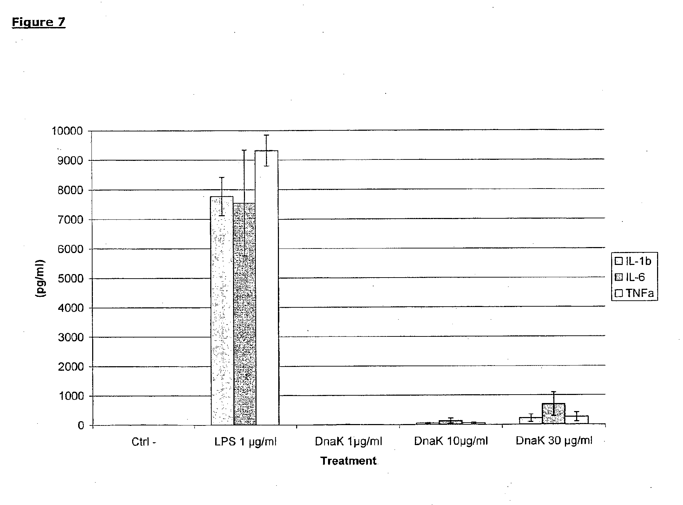

When added to whole blood, DnaK induced a very low production of interleukin 1β (IL-1β), interleukin 6 (IL-6) or Tumor Necrosis Factor α (TNFα), in comparison to bacterial lipopolysaccahrides (LPS). The production of these cytokines was detectable only at high concentration of DnaK (30 µg/ml) (figure 7). Cytokine production was quantitated in whole blood incubated 24 h in the presence of DnaK or LPS, the measurement was performed by ELISA.

### DnaK by itself does not induce T_{H}1 or T_{H}2 responses:

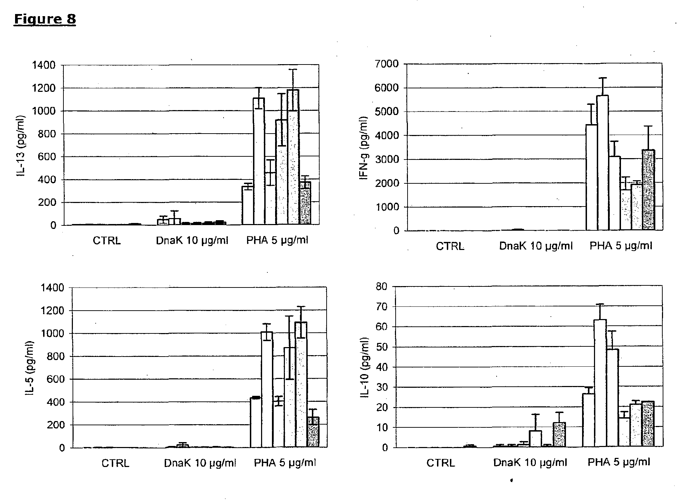

When human PBMC (peripheral blood lymphocytes) were incubated (6 days) in the presence of DnaK alone, there are no production of interferon-γ (IFN-γ) which would reflect the activation of T_{H}1 type lymphocytes (figure 8). The activation of T_{H}1 lymphocytes and production of IFN-γ lead to the activation of inflammation responses.

There are no production of interleukin 5 or 13 (IL-5, IL-13) (figure 8), which are produced following T_{H}2 lymphocyte activation. T_{H}2 responses are involved in the IgE antibodies production and mastocyte degranulation.

Positive control is PHA (phytohemagglutinin) which activate lymphocytes in a non-specific way. Cytokine production was measured by ELISA in the culture medium for 5 differents volunteers (each measurement was performed in duplicate).

A weak production of interleukin 10 (IL-10) was detectable in response to DnaK, in 2 samples from 6. IL-10 is involved in the tolerance phenomenon development.

### DnaK does not stimulate PBMC proliferation:

PBMC purified from human blood were incubated in 96 well plates for 5 days in the presence of DnaK (1-9 µg/ml) or Varicella zoster virus antigen (1 CPAU/ml as positive control). 100 µl of the medium were replaced by fresh medium containing 1 µCi of tritiated thymidine and the cells were further cultured for 16 hours. On day 6, incorporation of tritiated thymidin was measured with a beta counter using liquid scintillation.

| Condition | Cpm of [³H] thymidine |
|---|---|
| Buffer (negative control) | 2812 ± 3171 |
| DnaK (1 µg/ml) | 3334 ± 3441 |
| DnaK (3 µg/ml) | 3515 ± 3617 |
| DnaK (9 µg/ml) | 4405 ± 4601 |
| Varicella zoster antigens | 53408 ± 21180 |

Results are expressed as mean ± standard deviation of 5 determinations.

### DnaK inhibits IFN-γ production induced by another antigen:

Human PBMC were incubated 3 days with candidin, alone or in the presence of increasing concentrations of DnaK (1 - 20 µg/ml). IFN-γ in the culture medium was quantitated by ELISA. Results are presented as mean ± SD of 4 determinations.

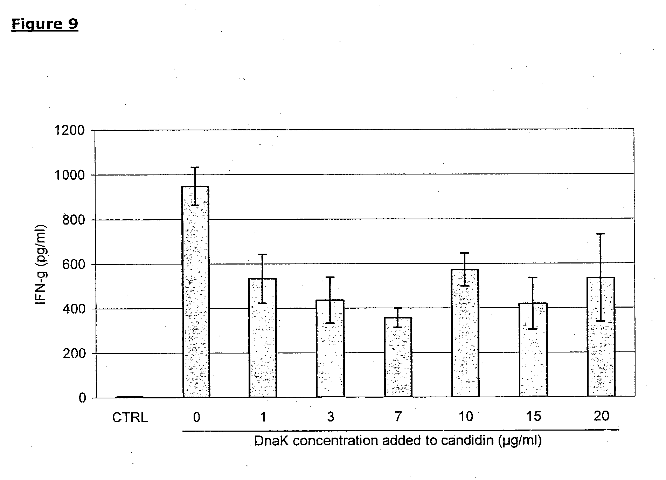

Since IFN-γ is involved in the inflammation response to antigens, the fact that its production was inhibited in the presence of DnaK suggest that DnaK could have some anti-inflammatory effect (figure 9).

### DnaK stimulates the production of IL-10 by Treg cells:

IL-10 is one mediator of the tolerance. It is produced by some Treg cells, which strongly expressed CD25 on their cell surface (they are CD25⁺ cells).

We have purified dendritic cells, CD25⁺ and CD25- cells from human blood and mixed them in precise ratio. Dendritic cells were first incubated in the presence of BSA or DnaK (10 µg/ml), then the lymphocytes were added: CD25- alone, CD25⁺ alone or the mixture CD25⁻/CD25⁺ (ratio 1:1). The culture was maintained for 3 days and the cells were stimulated or not with PHA.

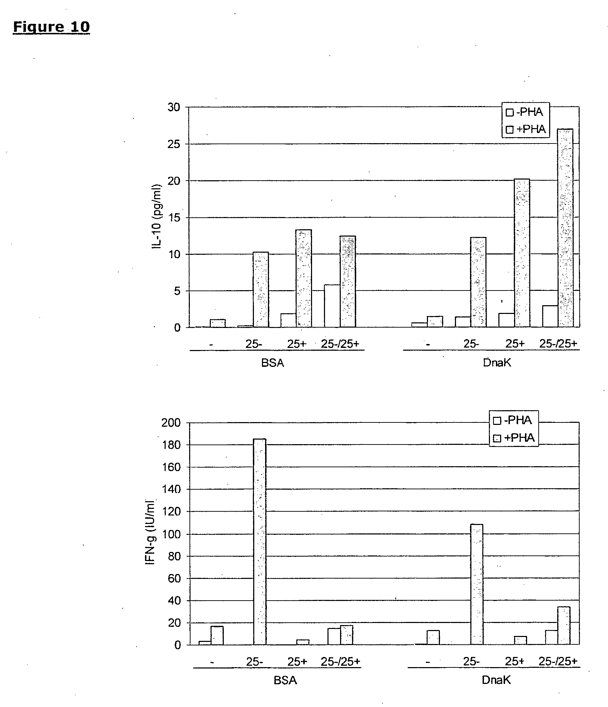

As expected, the presence of CD25⁺ cells in the culture inhibited the production of IFN-γ by CD25- cells (figure 10). The production of IL-10 by CD25⁺ cells is more important for cells incubated with DnaK versus cells incubated with BSA, the production is even increased for the mixutre of cell population. This suggest that DnaK stimulates the production of IL-10 by Treg, following the activation of T cells with PHA.

### There are anti-DnaK antibodies in human blood:

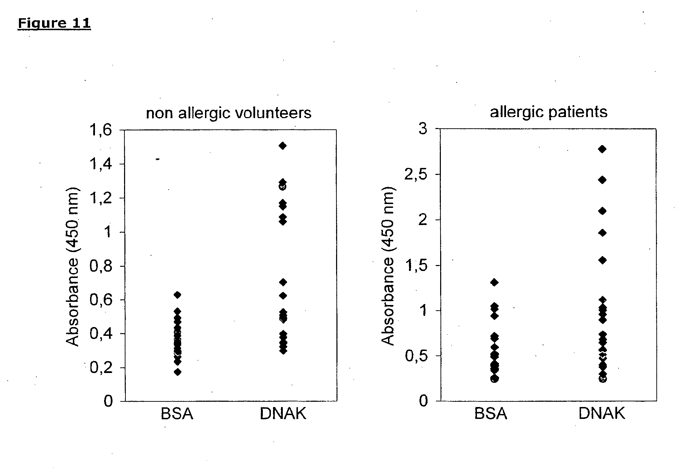

Using an ELISA test, we have pointed out the presence of anti-DnaK IgG in human serum. For 40% of the tested samples (from healthy volunteers and allergic patients), the OD₄₀₅ₙₘ was greater in the case of DnaK than with the BSA (figure 11) reflecting a higher titer of IgG against DnaK than against BSA.

## Claims

1. Recombinant purified DnaK preparation obtainable by a method comprising the steps of
a) ion exchange chromatography,
b) hydroxylapatite chromatography, and
c) gelatin chromatography,
wherein the Dnak preparation has:
- an ATPase activity without the addition of any other chaperone protein,
- a purity of 98 % by weight of protein, and
- is essentially free of T-cell stimulating impurities.

2. The recombinant purified DnaK preparation of claim 1 having
- a residual DNA contamination ≤ 1 ng/mg of protein,
- a residual host cell protein contamination (HCP) < 5% by weight, and
- an endotoxin contamination < 0.5 E.U./µg of protein.

3. The recombinant DnaK preparation of claim 1 or 2 wherein
- the residual DNA contamination is ≤ 0.5 ng/mg, and/or
- the HCP is < 0.1% by weight, and/or
- the endotoxin contamination is ≤ 0.01 E.U./µg of protein.

4. The recombinant DnaK preparation of any one of claims 1 to 3, wherein the DnaK is hydrolyzed.

5. The recombinant purified DnaK preparation of any one of claims 1 to 4, wherein the DnaK is
- in form of a complex with ADP,
- in form of a complex with ATP, or
- essentially free of nucleotides.

6. A method for the purification of a recombinant DnaK preparation as defined in one of claims 1 to 5 from a cell lysate comprising the steps of
a) ion exchange chromatography,
b) hydroxylapatite chromatography, and
c) gelatin chromatography.

7. The method of claim 6 wherein DnaK is from saprophytic bacteria, preferably *E. coli.*

8. The method of any one of claims 6 to 7 wherein
- the ion exchange chromatography is an anion exchange chromatography and/or
- wherein the hydroxylapatite chromatography is a hydroxylapatite type II chromatography.

9. The method of any one of claims 6 to 8 wherein the gelatin chromatography is conducted on gelatin sepharose.

10. A method for forming a complex between recombinant DnaK and at least one peptide or at least one protein comprising the steps of
a) combining recombinant DnaK of any one of claims 1 to 5 with ATP at molar ratio HSP:ATP of 1:1 to 1:10;
b) adding at least one peptide or at least one protein; and
c) incubating at a temperature of 10°C to 60°C, preferably 20°C to 45°C.

11. A mixture of recombinant DnaK as defined in claims 1 to 5 and at least one peptide or at least one protein, preferably in the form of a complex.

12. The DnaK of any one of claims 1 to 5 or the mixture or the complex of claim 11 for use in *in-vivo* diagnostics.

13. Use of the DnaK as defined in one of claims 1 to 5 or the mixture or the complex of claim 11 in *in-vitro* diagnostics.

14. The DnaK as defined in claims 1 to 5 or the mixture or complex of claim 11 for use in inducing tolerance, treatment or prevention of allergy, autoimmune diseases, graft rejection or neurodegenerative diseases or as an adjuvant inducing an humoral response or a regulatory T-cell response.

15. A pharmaceutical product comprising a recombinant purified DnaK preparation as defined in claims 1 to 5 or the mixture or complex of claim 11.

## Patentansprüche

1. Zubereitung von rekombinantem gereinigten DnaK, erhältlich nach einem Verfahren, das die folgenden Schritte umfasst:
a) Ionenaustauschchromatographie;
b) Hydroxylapatit-Chromatographie; und
c) Gelatine-Chromatographie;
wobei die DnaK-Zubereitung Folgendes aufweist:
- eine ATPase-Aktivität ohne Zugabe irgendeines anderen Chaperon-Proteins;
- eine Reinheit von 98 Gew.-% Protein; und
- im Wesentlichen frei von T-Zell-stimulierenden Verunreinigungen ist.

2. Zubereitung von rekombinantem gereinigten DnaK gemäß Anspruch 1, das Folgendes aufweist:
- eine Rest-DNA-Kontamination von ≤ 1 ng/mg Protein;
- eine Rest-Wirtszellprotein-Kontamination (HCP) von < 5 Gew.-%; und
- eine Endotoxin-Kontamination von < 0,5 E.U./µg Protein.

3. Zubereitung von rekombinantem DnaK gemäß Anspruch 1 oder 2, wobei
- die Rest-DNA-Kontamination ≤ 0,5 ng/mg ist; und/oder
- die HCP < 0,1 Gew.-% ist; und/oder
- die Endotoxin-Kontamination ≤ 0,01 E.U./µg Protein ist.

4. Zubereitung von rekombinantem DnaK gemäß einem der Ansprüche 1 bis 3, wobei das DnaK hydrolysiert ist.

5. Zubereitung von rekombinantem gereinigten DnaK gemäß einem der Ansprüche 1 bis 4, wobei das DnaK
- in Form eines Komplexes mit ADP vorliegt;
- in Form eines Komplexes mit ATP vorliegt; oder
- im Wesentlichen frei von Nucleotiden ist.

6. Verfahren zur Reinigung einer Zubereitung von rekombinantem DnaK, wie sie in einem der Ansprüche 1 bis 5 definiert ist, ausgehend von einem Zelllysat, umfassend die Schritte:
a) Ionenaustauschchromatographie;
b) Hydroxylapatit-Chromatographie; und
c) Gelatine-Chromatographie.

7. Verfahren gemäß Anspruch 6, wobei das DnaK aus saprophytischen Bakterien, vorzugsweise *E. coli,* stammt.

8. Verfahren gemäß einem der Ansprüche 6 bis 7, wobei
- die Ionenaustauschchromatographie eine Anionenaustauschchromatographie ist; und/oder
- wobei die Hydroxylapatit-Chromatographie eine Hydroxylapatit-Typ-II-Chromatographie ist.

9. Verfahren gemäß einem der Ansprüche 6 bis 8, wobei die Gelatine-Chromatographie auf Gelatine-Sepharose durchgeführt wird.

10. Verfahren zur Bildung eines Komplexes zwischen rekombinantem DnaK und wenigstens einem Peptid oder wenigstens einem Protein, umfassend die Schritte:
a) Kombinieren von rekombinantem DnaK gemäß einem der Ansprüche 1 bis 5 mit ATP in einem Stoffmengenverhältnis von HSP:ATP von 1:1 bis 1:10;
b) Hinzufügen wenigstens eines Peptids oder wenigstens eines Proteins; und
c) Inkubieren bei einer Temperatur von 10 °C bis 60 °C, vorzugsweise 20 °C bis 45 °C.

11. Gemisch von rekombinantem DnaK, wie es in den Ansprüchen 1 bis 5 definiert ist, und wenigstens einem Peptid oder wenigstens einem Protein, vorzugsweise in Form eines Komplexes.

12. DnaK gemäß einem der Ansprüche 1 bis 5 oder Gemisch oder Komplex gemäß Anspruch 11 zur Verwendung in der in-vivo-Diagnose.

13. Verwendung des DnaK gemäß einem der Ansprüche 1 bis 5 oder des Gemischs oder Komplexes gemäß Anspruch 11 bei der in-vitro-Diagnose.

14. DnaK gemäß einem der Ansprüche 1 bis 5 oder Gemisch oder Komplex gemäß Anspruch 11 zur Verwendung bei der Induktion einer Toleranz gegenüber, bei der Behandlung oder Prävention einer Allergie, von Autoimmunkrankheiten, von Transplantatsabstoßung oder neurodegenerativen Erkrankungen oder als Adjuvans, das eine humorale Antwort oder eine regulatorische T-Zell-Antwort induziert.

15. Pharmazeutisches Produkt, umfassend eine Zubereitung von rekombinantem gereinigten DnaK, wie es in den Ansprüchen 1 bis 5 definiert ist, oder das Gemisch oder den Komplex gemäß Anspruch 11.

## Revendications

1. Préparation de DnaK purifiée recombinante que l'on peut obtenir par un procédé comprenant les étapes de
a) chromatographie par échange d'ions,
b) chromatographie sur hydroxyapatite, et
c) chromatographie sur gélatine,
dans laquelle la préparation de DnaK a :
- une activité d'ATPase sans addition de toute autre protéine chaperon,
- une pureté de 98 % en poids de protéine, et
- est essentiellement exempte d'impuretés de stimulation de cellules T.

2. Préparation de DnaK purifiée recombinante selon la revendication 1, ayant
- une contamination par ADN résiduel ≤ 1 ng/mg de protéine,
- une contamination par protéine de cellule hôte résiduelle (HCP) < 5 % en poids, et
- une contamination par endotoxine < 0,5 U.E./µg de protéine.

3. Préparation de DnaK recombinante selon la revendication 1 ou 2, dans laquelle
- la contamination par ADN résiduel est ≤ 0,5 ng/mg, et/ou
- la HCP est < 0,1 % en poids, et/ou
- la contamination par endotoxine est ≤ 0,01 U.E./µg de protéine.

4. Préparation de DnaK purifiée recombinante selon l'une quelconque des revendications 1 à 3, dans laquelle la DnaK est hydrolysée.

5. Préparation de DnaK recombinante purifiée selon l'une quelconque des revendications 1 à 4, dans laquelle la DnaK est
- sous forme d'un complexe avec ADP,
- sous forme d'un complexe avec ATP, ou
- essentiellement exempte de nucléotides.

6. Procédé de purification d'une préparation de DnaK recombinante telle que définie dans l'une des revendications 1 à 5 à partir d'un lysat cellulaire comprenant les étapes de :
a) chromatographie par échange d'ions,
b) chromatographie sur hydroxyapatite, et
c) chromatographie sur gélatine.

7. Procédé selon la revendication 6, dans lequel la DnaK provient de bactéries saprophytes, de préférence *E. coli.*

8. Procédé selon la revendication 6 ou 7, dans lequel
- la chromatographie par échange d'ions est une chromatographie par échange d'anions et/ou
- dans lequel la chromatographie sur hydroxyapatite est une chromatographie de type II sur hydroxyapatite.

9. Procédé selon l'une quelconque des revendications 6 à 8, dans lequel la chromatographie sur gélatine est menée sur un gel de sépharose.

10. Procédé de formation d'un complexe entre une DnaK recombinante et au moins un peptide ou au moins une protéine comprenant les étapes de :
a) combinaison de la DnaK recombinante de l'une quelconque des revendications 1 à 5 avec de l'ATP à un rapport molaire HSP : ATP de 1 : 1 à 1 : 10 ;
b) ajout d'au moins un peptide ou d'au moins une protéine ; et
c) incubation à une température de 10 °C à 60 °c, de préférence 20 °C à 45 °C.

11. Mélange de Dnak recombinante telle que définie dans les revendications 1 à 5 et d'au moins un peptide ou d'au moins une protéine, de préférence sous forme d'un complexe.

12. DnaK selon l'une quelconque des revendications 1 à 5 ou mélange ou complexe de la revendication 11 destiné à être utilisé dans des diagnostics *in vivo.*

13. Utilisation de la DnaK telle que définie dans l'une des revendications 1 à 5 ou mélange ou complexe de la revendication 11 dans des diagnostics *in vitro.*

14. DnaK telle que définie dans les revendications 1 à 5 ou mélange ou complexe selon la revendication 11, pour utilisation pour induire une tolérance, un traitement ou une prévention d'une allergie, de maladies auto-immunes, d'un rejet de greffe ou de maladies neurodégénératives ou en tant qu'adjuvant induisant une réponse humorale ou une réponse de cellule T régulatrice.

15. Produit pharmaceutique comprenant une préparation de DnaK purifiée recombinante telle que définie dans les revendications 1 à 5 ou mélange ou complexe de la revendication 11.
